(19) Europäisches Patentamt
European Patent Office
Office européen des brevets

(11) Veröffentlichungsnummer: **0 055 801**
**B1**

(12) **EUROPÄISCHE PATENTSCHRIFT**

(45) Veröffentlichungstag der Patentschrift: **31.10.84**

(21) Anmeldenummer: **81108305.4**

(22) Anmeldetag: **14.10.81**

(51) Int. Cl.³: **B 01 J 19/06**, C 23 F 7/08, C 23 G 1/02, C 09 D 7/00, A 61 K 7/00, C 11 D 3/37, C 08 F 20/00, D 06 M 15/30

(54) **Verfahren zum Verdicken von wässrigen Systemen.**

(30) Priorität: **24.12.80 DE 3049178**

(43) Veröffentlichungstag der Anmeldung: **14.07.82 Patentblatt 82/28**

(45) Bekanntmachung des Hinweises auf die Patenterteilung: **31.10.84 Patentblatt 84/44**

(84) Benannte Vertragsstaaten: **BE DE FR GB IT NL SE**

(56) Entgegenhaltungen:
**EP - A - 0 027 850**
**DE - B - 1 082 004**
**FR - A - 1 280 411**
**GB - A - 2 043 081**
**US - A - 3 575 881**

**Die Akte enthält technische Angaben, die nach dem Eingang der Anmeldung eingereicht wurden und die *nicht in dieser Patentschrift enthalten sind.***

(73) Patentinhaber: **Röhm GmbH, Kirschenallee Postfach 4242, D-6100 Darmstadt 1 (DE)**

(72) Erfinder: **Fink, Herbert, Dr., Berliner Strasse 24, D-6101 Bickenbach (DE)**
Erfinder: **Hübner, Klaus, Dr., Leipziger Strasse 9, D-6105 Ober-Ramstadt-Eiche (DE)**
Erfinder: **Markert, Gerhard, Dr., Leipziger Strasse 25, D-6105 Ober-Ramstadt-Eiche (DE)**
Erfinder: **Sütterlin, Norbert, Dr., Am Horeth 23, D-6105 Ober-Ramstadt (DE)**

**EP 0 055 801 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft ein Verfahren zum Verdicken von wäßrigen Systemen gemäß dem Oberbegriff des Anspruchs 1. Kennzeichnend für wäßrige Dispersionen, die als Verdickungsmittel eingesetzt werden, ist ein Gehalt an salzbildenden Gruppen in dem dispergierten synthetischen Polymeren und die niedrige Viskosität der daraus gebildeten wäßrigen Dispersionen im neutralen Bereich. Beispielsweise enthalten die Emulsionspolymerisate gemäß der DE-AS 1 109 132 10 bis 30% an Acryl- oder Methacrylsäureeinheiten neben Einheiten von Acryl- oder Methacrylsäureestern. Bei Zusatz einer Base geht die dünnflüssige, milchige Dispersion in eine dickflüssige wäßrige Lösung über. Wäßrige Systeme lassen sich auf einfache Weise verdicken, indem man sie mit einer dünnflüssigen Dispersion dieser Art vermischt und durch Zugabe einer Base auf einen pH-Wert im alkalischen Bereich einstellt, bei welchem das carboxylgruppenhaltige Polymere in die Salzform übergeht und wenigstens kolloidal löslich ist.

Es ist allerdings nicht immer erwünscht, daß das verdickte wäßrige System alkalisch ist. Zum Beispiel werden wäßrige Kunststoffdispersionen für die Textilausrüstung gern in verdickter Form angewendet. Sie müssen sauer eingestellt sein, wenn sie selbstvernetzende Gruppen oder Kondensationsharze enthalten, die nur im sauren Bereich vernetzen. In diesen Fällen konnte man Dispersionen carboxylgruppenhaltiger Polymere nicht als Verdickungsmittel einsetzen.

Die Erfinder haben sich die Aufgabe gestellt, ein Verfahren zum Verdicken von wäßrigen Systemen zu entwickeln, das auch dann anwendbar ist, wenn das verdickte wäßrige System sauer ist. Eine ältere Lösung der gestellten Aufgabe besteht in der Verwendung von Emulsionspolymerisaten gemäß EP-B 27 850, die hier ausgeschlossen ist. Die vorliegende Erfindung betrifft ein Verfahren zum Verdicken von wäßrigen Systemen durch Vermischen des wäßrigen Systems mit einer wäßrigen Dispersion eines synthetischen Polymeren, das aus

A) 5—100 Gew.-% eines ungesättigten, radikalisch polymerisierbaren Monomeren mit wenigstens einem basischen Stickstoffatom,

B) 0— 95 Gew.-% eines in Wasser höchstens begrenzt löslichen, ungesättigten, radikalisch polymerisierbaren Comonomeren,

C) 0— 30 Gew.-% eines in Wasser löslichen, nicht basischen ungesättigten, radikalisch polymerisierbaren Comonomeren

aufgebaut ist und ein Molekulargewicht von mindestens 500.000 hat und bei einem unter 7 liegenden pH-Wert wenigstens kolloidal löslich ist, ausgenommen Emulsionspolymerisate von 3-Dialkylamino-2,2-dimethylpropylestern einer ungesättigten polymerisierbaren Carbonsäure, sowie das Mischpolymerisat aus 30% Dimethylaminoäthylmethacrylat, 30% Äthylacrylat und 40% Methylcrylat. Erfindungsgemäß wird das Gemisch bei einem pH-Wert unter 7 verdickt. Das letztgenannte Emulsionspolymerisat wirkt zwar als Verdickungsmittel im sauren Bereich, neigt aber schon beim Stehen ohne Ansäuern zum Verdicken, hat also nur eine begrenzte Lagerfähigkeit.

Da dies ein Nachteil aller Emulsionspolymerisate des erfindungsgemäß eingesetzten Typs ist, sofern sie als Monomerkomponente A mit einem basischen Stickstoffatom überwiegend oder ausschließlich Einheiten von Dimethylaminoäthylmethacrylat enthalten, werden diese Dispersionen bei der bevorzugten Ausführungsform der Erfindung nicht verwendet.

Es sind zwar verschiedene Polymere von ungesättigten Monomeren mit einem Stickstoffatom bekannt, jedoch entsrechen sie aus verschiedenen Gründen nicht den erfindungsgemäß eingesetzten synthetischen Polymeren.

US-A 3 575 881 betrifft Polymere mit Amidgruppen, abgeleitet von Monomeren mit der Strukturgruppe

$$CH_2{=}CH{-}\overset{|}{N}{-}CO{-}.$$

Das Stickstoffatom in der Amidgruppe ist nicht basisch. Infolgedessen erleiden die bekannten Polymeren keine Veränderung ihrer Löslichkeit bei pH-Werten unter 7. Es handelt sich daher weder vom Aufbau noch von den Eigenschaften her um Polymere gemäß der vorliegenden Erfindung.

FR-A 1 280 411 betrifft Verdickungsmittel für wäßrige Systeme, bestehend aus Polyacrylamid bzw. -N-butylamid. Auch dieses Polymere enthält keine basischen Stickstoffatome, sondern nur nichtbasische Stickstoffatome in Amidgruppierungen.

GB-A 2 043 081 betrifft stark vernetzte Perlpolymerisate, hergestellt durch Polymerisation einer wäßrigen Monomerenlösung, die in einer Ölphase zu Tröpfchen verteilt ist, zu Polymerisatperlen mit einem Durchmesser von 100—200 μm.

Demnach handelt es sich nicht um eine wäßrige Dispersion, wie erfindungsgemäß verwendet. Die Teilchen sind wesentlich größer als in wäßrigen Dispersionen und sind wegen der starken Vernetzung bei jedem pH-Wert in Wasser unlöslich.

DE-B 1 082 004 betrifft Polymere von Monomeren, die quartäre Ammoniumgruppen enthalten. Quartäre Ammoniumgruppen sind nicht basisch. Sie liegen vielmehr bei jedem pH-Wert in der Salzform

vor und haben bei jedem pH-Wert die gleichen Löslichkeitseigenschaften. Die Polymeren gemäß der Entgegenhaltung liegen nicht als wäßrige Dispersion vor.

Die Verdickungswirkung der erfindungsgemäß eingesetzten Dispersionen beruht auf den basischen Stickstoffatomen der Monomerkomponente A. Im sauren Bereich liegen diese Stickstoffatome in Form der entsprechenden Ammoniumsalze vor. Die Hydrophilie der Ammoniumsalze ist bedeutend größer als die der zugrundeliegenden Aminogruppe und erhöht die Hydrophilie des gesamtenn Polymerisats. Der Eintritt der Verdickungswirkung hängt von einer Reihe von Bedingungen ab, die nachfolgend zusammengestellt werden.

1. In der Komponente A muß wenigstens ein Stickstoffatom vorhanden sein, das im sauren Bereich in die Salzform übergeht. Wenn diese Voraussetzung erfüllt ist, handelt es sich um ein »basisches Stickstoffatom« im Sinne der Erfindung. Der $pK_b$-Wert des zugrundeliegenden Monomeren soll vorzugsweise im Bereich von 3 bis 6 liegen.
2. Das synthetische Polymere muß in der Basenform so wenig hydrophil sein, daß es in der wäßrigen Phase der Dispersion unlöslich und höchstens leicht mit Wasser gequollen ist, da es anderenfalls nicht im dispergierten Zustand vorliegen könnte.
3. Das synthetische Polymere muß im sauren Bereich nach Überführung in die Salzform so stark hydrophil sein, daß es echt gelöst oder wenigstens kolloidal gelöst ist.
   Die kolloidalen Lösungen unterscheiden sich von echten Lösungen äußerlich durch einen schwachen Tydall-Effekt, d. h. eine leichte Streuung des durchfallenden Lichtes, und physikalisch durch unvollständige Hydratisierung und Streckung der Makromoleküle. Kolloidal gelöste Poylmerisate liegen im wesentlichen in Form stark gequollener, submikroskopischer Gelpartikel vor.
4. Das Polymerisat in der Salzform muß im echt oder kolloidal gelösten Zustand eine Verdickungswirkung besitzen, wozu ein Molekulargewicht von mindestens 500.000 und vorzugsweise mehr als 1 Million erforderlich ist. Besonders vorteilhaft ist ein stark verzweigter Aufbau, der bis an die Grenze der Vernetzung führen kann, was durch den Einbau geringer Mengen an Vernetzungsmitteln erreichbar ist. Mit zunehmender Verzweigung oder Vernetzung geht der Übergang von echt gelösten zum kolloidal gelösten Zustand einher. Bei zu starker Vernetzung gehen die Quellbarkeit und kolloidale Löslichkeit verloren.

Polymerdispersionen, die alle genannten Forderungen erfüllen, sind im neutralen bis alkalischen Bereich milchartig und dünnflüssig und im sauren Bereich hochviskos und klar. Sie können mit einem zu verdickenden wäßrigen System, das bereits sauer ist, vermischt werden und verdicken dann innerhalb von wenigen Sekunden oder Minuten. Im allgemeinen wird die gegebenenfalls vorher verdünnte wäßrige Dispersion in das wäßrige System eingerührt; man kann aber auch umgekehrt verfahren. Wenn das zu verdickende wäßrige System beim Vermischen mit der Dispersion nicht sauer ist, behält es seine Viskosität zunächst bei und verdickt erst beim Ansäuern.

Als wäßrige Systeme werden im Sinne der Erfindung reines Wasser und alle flüssigen Mischungen, die Wasser als wesentliche Komponente enthalten, angesehen. Im allgemeinen enthalten sie wenigstens 20 Gew.-% und vorzugsweise mehr als 50 Gew.-% Wasser. Neben Wasser können auch andere flüssige Bestandteile in dem wäßrigen System enthalten sein, vor allem wassermischbare organische Flüssigkeiten, wie niedere Alkohole, Glykole, Glycerin, Ketone, niedere Carbonsäure, Formamide, Acetamide, Dimethylsulfoxid, Tetrahydrofuran, Dioxin u. a. Die wäßrige Phase kann auch organische oder anorganische Festsubstanzen gelösten enthalten, wie z. B. Harnstoff, Zucker, Seifen, Emulgatoren, anorganische Salze oder Säuren, makromolekulare Stoffe, wie Eiweiße, Celluloseäther oder synthetische wasserlösliche Polymere.

Das wäßrige System kann wasserunlösliche Bestandteile enthalten und mit diesen wäßrige Dispersionen, Suspensionen, Emulsionen oder Kolloide bilden. Wäßrige Kunststoffdispersionen sind ein bevorzugtes Anwendungsgebiet der Erfindung. Das wäßrige System kann auch seinerseits in einem nicht-wäßrigen Medium emulgiert sein. In diesem Falle läßt sich nur das emulgierte wäßrige System innerhalb der emulgierten Tröpfchen erfindungsgemäß verdicken, jedoch im allgemeinen nicht die nicht-wäßrige Emulsion als solche.

Als Verdickung wird jeder merkliche Anstieg der Viskosität des wäßrigen Systems bezeichnet. In manchen Fällen genügt ein geringer Viskositätsanstieg, z. B. um ein turbulent strömendes wäßriges Medium zu laminarer Strömung zu veranlassen. Das wäßrige System kann schon in einem verdickten Ausgangszustand vorliegen und erfindungsgemäß zu einer noch höheren Viskosität verdickt werden. Im allgemeinen spricht man von einer Verdickung erst dann, wenn die meßbare Viskosität um wenigstens 10% über den Ausgangswert ansteigt. Bei niedrigen Ausgangsviskositäten ist die Verdickung meistens erst dann spürbar und von anwendungstechnischer Bedeutung, wenn das Produkt aus der Ausgangsviskosität, gemessen in mPa s, und dem in Prozent ausgedrückten Viskositätsanstieg den Wert von 1000 erreicht oder überschreitet. Dies wird an einigen Zahlenbeispielen erläutert:

| Ausgangsviskosität | Endviskosität | prozentualer Visk.-anstieg | Produkt Ausgangs-Visk. × Visk.-anstieg |
|---|---|---|---|
| 1 mPa s | 11 mPa s | 1000% | 1 000 mPa s % |
| 10 mPa s | 20 mPa s | 100% | 1 000 mPa s % |
| 100 mPa s | 110 mPa s | 10% | 1 000 mPa s % |
| 1000 mPa s | 1100 mPa s | 10% | 10 000 mPa s % |

Im Regelfall wird eine Endviskosität von wenigstens 100 mPa s angestrebt. Typische verdickte, wäßrige Systeme haben Viskositätswerte im Bereich von 1000 bis 15.000 mPa s bei Raumtemperatur. Im Gegensatz zu den erfindungsgemäß verdickten wäßrigen Systemen stehen solche, die zum Zwecke der Flockung oder Sedimentationsbeschleunigung geringe Mengen an wasserlöslichen Polymeren enthalten, die keine merkliche Viskositätserhöhung bewirken.

Die zum Verdicken erforderliche Menge des dispergierten Polymeren hängt von seiner Wirksamkeit und der angestrebten Endviskosität ab. Sie liegt häufig im Bereich von 0,5 bis 10 Gew.-%, und bevorzugt im Bereich von 1 bis 3 Gew.-%, berechnet auf reines Polymerisat und bezogen auf das Gewicht des Wasseranteils in dem wäßrigen System. In manchen Fällen tritt nach Zusatz der wäßrigen Dispersion die Verdickung des wäßrigen Systems, auch wenn es sauer eingestellt ist, noch nicht bei Raumtemperatur, sondern erst beim Erwärmen ein. Beim Abkühlen bleibt die in der Wärme erreichte Verdickung im allgemeinen bestehen.

Die Verdickung tritt bei einem pH-Wert ein, bei dem die in dem synthetischen Polymeren enthaltenen basischen Stickstoffatome in die Ammoniumsalzform übergehen. Im Regelfall geschieht das bereits bei pH-Werten dicht unter 7. Infolge einer gewissen Pufferwirkung des Polymeren tritt die volle Verdickungswirkung erst bei pH-Werten unter 5 ein. Unterhalb pH 3 wird im allgemeinen keine weitere Verdickung mehr bewirkt. Der in der Praxis überwiegend angewandte pH-Bereich der verdickten wäßrigen Systeme liegt zwischen pH 2,0 und 5,5.

Die Monomerkomponente A des synthetischen Polymeren muß wenigstens eine zur radikalischen Polymerisation oder zumindest Copolymerisation befähigte Kohlenstoff-Doppelbindung, insbesondere in Form einer Vinyl-, Vinyliden- oder Vinylgruppe, und wenigstens ein basisches Stickstoffatom in Form einer sekundären oder tertiären Aminogruppe, die gegebenenfalls auch Bestandteil eines Ringsystems sein kann, enthalten. Primäre Aminogruppen sind im allgemeinen weniger geeignet als sekundäre und vor allem tertiäre Aminogruppen, weil sie die Polymerisation stören. Beispiele geeigneter Monomerer sind N-Vinylimidazol, -imidazolin, -imidazolidin, 4-Vinylpyridin und die N-substituierten Amide von $\alpha,\beta$-ungesättigten polymerisierbaren Mono- oder Dicarbonsäuren, die wenigstens ein basisches Stickstoffatom im N-Substituenten enthalten.

Wegen ihrer ausgeprägten Verdickungswirkung sind die Ester von $\alpha,\beta$-ungesättigten polymerisierbaren Mono- oder Dicarbonsäuren, die wenigstens ein basisches Stickstoffatom im Alkoholrest enthalten, als Komponente A bevorzugt. Die Acryl- und Methacrylester sind besonders bevorzugt. Weiterhin seien Ester von Malein-, Fumar- und Itakonsäure genannt. Die erwähnten Ester enthalten die Struktur

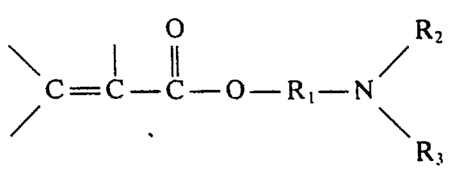

wobei die Gruppen $R_1$, $R_2$ und $R_3$ zusammengenommen vorzugsweise mindestens 5 C-Atome enthalten und $R_2$ und $R_3$ zusammen mit dem Stickstoffatom ein heterozyklisches Ringsystem bilden können. Diejenigen Ester, die als $R_1$ eine Äthylengruppe enthalten, ergeben Dispersionen, die schon oberhalb pH 7 zum Verdicken neigen, und zwar umso mehr, je kleiner der gesamte Alkoholrest des Esters ist. Deshalb gehören die Dispersionen von Polymerisaten des nur 4 C-Atome im Alkoholrest enthaltende Dimethylaminoäthylmethacrylats nicht zu den bevorzugten Ausführungsformen der Erfindung. Ester der obigen Struktur mit 5 oder mehr C-Atomen leiten sich z. B. vom N,N-Diäthylaminoäthanol, N-Butylaminoäthanol, N,N-Dipropylaminoäthanol, 2-Piperidino-äthanol, 2-(4-Morpholinyl)-äthanol, 2(N-Imidazolyl)-äthanol oder 2-Piperazinoäthanol ab. Vorzugsweise hat die Gruppe $R_1$ wenigstens 3 C-Atome, wie z. B. im Falle der Ester des 2-Dimethylamino-1-propanols oder Dimethylamino-isopropanols und besonders bevorzugt wenigstens 3 C-Atome in gerader Kette zwischen dem Ester-Sauerstoffatom und dem basischen Stickstoffatom. Beispiele dieser besonders bevorzugten Ester sind 3-Dimethyl(äthyl)aminopropylacrylat und -methacrylat und 4-Dimethylaminobutylacrylat und

-methacrylat. Die Emulsionspolymerisate gemäß EP-B 27 850 enthalten ebenfalls Einheiten von Estern der obigen Struktur, sind jedoch aus dem Schutzumfang ausgenommen.

Homopolyrisate der Monomeren A sind als synthetische Polymere für die Erfindung geeignet, sofern sie die weiter oben aufgezählten Voraussetzungen erfüllen. Das ist aber nicht immer der Fall, z. B. wenn die Homopolymerisate schon im alkalischen Bereich wasserlöslich sind oder $T_{\Lambda\,max}$-Werte über 100°C haben.

Man verwendet daher häufig Copolymerisate, die neben der Komponente A Einheiten von schwach wasserlöslichen Monomeren B oder von gut wasserlöslichen Monomeren C oder von beiden enthalten. Als in Wasser höchstens begrenzt lösliche Monomere werden solche mit einer Wasserlöslichkeit von höchstens 10% bei 20°C angesehen. Ihr Anteil an dem synthetischen Polymeren kann 95 Gew.-% erreichen und beträgt vorzugsweise 50 bis 80 Gew.-%. Hierzu gehören die Alkylester der Acryl- und Methacrylsäure, wie z. B. die Methyl-, Äthyl-, n-Propyl-, Isopropyl-, n-Butyl-, sec. Butyl-, Hexyl-, Cyclohexyl-, 2-Äthylhexyl-, Octyl- und Dodecylester, Vinylester, wie Vinylacetat, -propionat, -butyrat und -hexoat, Vinyläther, wie Methyl-, Äthyl- oder Butylvinyläther, Styrol, Acryl- und Methacrylnitril, Äthylen, Propylen usw.

Die Hydrophilie des Emulsionspolymerisats läßt sich erforderlichenfalls durch untergeordnete Mengen, die nicht mehr als 30 Gew.-% des synthetischen Polymeren ausmachen, an nicht basischen wasserlöslichen Comonomeren C erhöhen. Sie haben eine Wasserlöslichkeit von mehr als 10 Gew.-% bei 20°C. Dazu gehören z. B. Hydroxyäthylacrylat oder -methacrylat, Acryl- oder Methacrylamid, Vinylpyrrolidon sowie Comonomere mit quartären Ammoniumgruppen, wie Methacryloxyäthyl-trimethylammoniumchlorid. Comonomere mit Carboxyl- bzw. Carboxylatgruppen, wie Acryl- oder Methacrylsäure, Malein-, Fumar- oder Itakonsäure und deren wasserlösliche Salze, können sich in manchen Fällen durch Wechselwirkung mit den basischen Stickstoffatomen nachteilig auf die Verdickungseigenschaften auswirken. Wenn ihre Anwesenheit aus besonderen Gründen erwünscht ist, beispielsweise zur Verbesserung der Stabilität, so muß im Einzelfall die Verträglichkeit mit dem Gesamtsystem überprüft werden.

Die Monomeren der Gruppen A, B und C können nicht willkürlich gewählt werden, sondern es bedarf einer sorgfältigen gegenseitigen Abstimmung, um die oben genannten Voraussetzungen zu erfüllen. Das gilt vor allem für die Einstellung der Gesamthydrophilie im sauren und alkalischen Bereich und der dynamischen Einfriertemperatur $T_{\Lambda\,max}$. Die Monomeren A sind im allgemeinen verhältnismäßig hydrophil; ihre Wasserlöslichkeit bei 20°C kann über oder unter 10% liegen. Ein hoher Anteil dieser Monomeren ruft eine starke Verdickungswirkung hervor.

Ähnlich wie die Monomeren der Gruppe A erhöhen die wasserlöslichen Comonomeren C die Hydrophilie des Polymeren und zwar umso stärker, je höher ihre Wasserlöslichkeit ist. Die Monomeren der Gruppe B vermindern dagegen die Hydrophilie. Diese Wirkung ist umso stärker ausgeprägt, je mehr und je größere hydrophobe Gruppen in dem Monomeren enthalten sind. Aliphatische Reste mit 4 oder mehr Kohlenstoffatomen sowie aromatische Gruppen wirken stark hydrophobierend. Träger solcher Gruppen sind z. B. Butyl-, Hexyl- oder Octylester ungesättigter Carbonsäuren und Styrol. Dagegen wirken die entsprechenden Methyl-, Äthyl- und Propylester sowie Vinylester der niederen Fettsäuren und Acryl- oder Methacrylnitril nur schwach hydrophobierend.

Die dynamische Einfriertemperatur $T_{\Lambda\,max}$ (nach DIN 53 445) läßt sich in bekannter Weise durch die Wahl des Mischungsverhältnisses von »harten« und »weichen« Monomeren sehr genau einstellen und soll im allgemeinen unter 100°C liegen. »Harte« Monomere sind solche, die Homopolymerisate mit hoher dynamischer Einfriertemperatur bzw. mit hoher Glastemperatur bilden. Von den unter die Komponente A fallenden Monomeren gehören dazu diejenigen mit einer Amidstruktur. Harte Monomere der Gruppe B sind vor allem die niederen Methacrylester, Acryl- und Methacrylnitril und Styrol und aus der Gruppe C die Amide der Acryl- und Methacrylsäure sowie — falls verwendbar — diese Säuren selbst. Unter den weichen Monomeren sind insbesondere die niederen Acrylester, nämlich Methyl-, Äthyl- und n-Propylacrylat zu nennen, weil sie zugleich wenig hydrophob sind und die Herstellung weicher, hydrophiler Mischpolymerisate gestatten.

Andere weiche Comonomere, wie die höheren Ester der Acrylsäure und Methacrylsäure, von den Butylestern aufwärts, setzen gleichzeitig die Erweichungstemperatur und die Hydrophilie herab. Synthetische Polymere mit einer dynamischen Einfriertemperatur unter 20°C haben bei Raumtemperatur eine hohe Verdickungswirkung und sind deshalb bevorzugt.

H. Weßlau hat in »Makromolekulare Chemie«, Band 69 (1963), Seiten 220 bis 240, die Abhängigkeit der Verdickungswirkung von der Erweichungs- bzw. Einfriertemperatur einerseits und der Hydrophilie des Polymerisats andererseits beschrieben und die Beeinflussung dieser Größen durch die Wahl der Comonomeren erläutert.

Die »Härte« von zahlreichen Monomeren, ausgedrückt durch die Glastemperatur des Homopolymerisats, ist im »Polymer Handbook« von J. Brandrup und E. H. Immergut (1975, Seiten III/139—192) angegeben. Die Abstimmung der Polymerisateigenschaften durch die Abmischung von verschiedenen Comonomeren gelingt umso besser, je ähnlicher ihr Polymerisationsverhalten ist. Auch hierzu finden sich in dem erwähnten »Polymer Handbook« Zahlenwerte (Q,e) für viele Monomere (Seiten II/387 bis 404). Besonders vorteilhaft verhalten sich in dieser Hinsicht die Abkömmlinge der Acryl- und Methacrylsäure, so daß es empfehlenswert ist, die Monomeren aller drei Gruppen A, B und C ganz oder zum

überwiegenden Teil unter diesen Abkömmlingen auszuwählen.

Da die Verdickungswirkung mit steigendem Molekulargewicht des synthetischen Polymeren zunimmt, sollten alle Einflüsse, die sich erniedrigend auf das Molekulargewicht auswirken, weitmöglichst ausgeschaltet werden; kettenübertragend wirkende Zusätze und ein zu hoher Radikalstrom bei der Polymerisation sollten vermieden werden. Die Verdickungswirkung wird durch geringe Anteile von Vernetzungsmitteln gefördert. Darunter werden Monomere mit zwei oder mehr radikalisch polymerisierbaren Gruppen verstanden. Sie sind vorzugsweise in Mengen von 0,01 bis 0,5 Gew.-% am Aufbau der synthetischen Polymeren beteiligt. Beispiele für geeignete Vernetzungsmittel sind Äthylenglykoldiacrylat oder -dimethacrylat, 1,2-Propylenglykoldiacrylat oder -dimethacrylat, Butandiol-1,4-diacrylat oder -dimethacrylat, Methylen-bis acrylamid oder -methacrylamid und Divinylbenzol.

Die synthetischen Polymeren lassen sich nach den bekannten Methoden der wäßrigen Emulsionspolymerisation herstellen. Als Emulgiermittel finden die üblichen Emulgatoren mit nicht-ionischen, kationischen, anionischen oder amphoteren Eigenschaften in den üblichen Mengen, die beispielsweise zwischen 0,01 und 10 Gew.-% der wäßrigen Phase liegen, Verwendung. Eine eventuelle störende Wechselwirkung zwischen den basischen Stickstoffatomen der Monomerkomponente A und anionischen Emulgatoren läßt sich durch die Verminderung des Anteils an anionischen Emulgatoren oder die Verwendung von kationischen oder nichtionischen Emulgatoren oder deren Gemischen vermeiden. Als Beispiele für verwendbare Emulgatoren seien Natriumlaurylsulfat, Natriumdodecylbenzolsulfonat, Stearyldimethyl-benzylammoniumchlorid, Cocosfettaminhydrochlorid, Oxäthylierungsprodukte von Alkylphenolen, Fettalkoholen oder Fettsäuren mit etwa 20—100 Mol Äthylenoxideinheiten und Amphotenside genannt.

Man kann zur Herstellung der Emulsionspolymerisate die Monomeren in emulgierter Form mit den erforderlichen Hilfsstoffen vorlegen und die Polymerisation einleiten. Es wird jedoch im allgemeinen bevorzugt, die Monomeren als solche oder in Form einer wäßrigen Emulsion im Laufe des Polymerisationsverfahrens allmählich zulaufen zu lassen. Zur Auslösung der Polymerisation werden die bekannten radikalbildenden Initiatoren in den gebräuchlichen Mengen verwendet. Wasserlösliche Azoinitiatoren, wie Alkalisalze der Azo-bis-cyanvaleriansäure, sind besonders vorteilhaft. Bei Polymerisationstemperaturen, die beispielsweise zwischen 40 und 100°C liegen, lassen sich stabile Dispersionen mit Feststoffgehalten bis zu 60 Gew.-% herstellen. Vorzugsweise liegt der Feststoffgehalt zwischen 30 und 60 Gew.-%. Die Dispersionen haben ein milchig weißes Aussehen und reagieren neutral bis schwach basisch. Ihre Viskosität beträgt in typischen Fällen von 20 bis 5000 mPa s.

Das zu verdickende wäßrige System kann bereits sauer sein und verdickt dann rasch nach dem Vermischen mit der Dispersion. Im allgemeinen wird es vorgezogen, das wäßrige System bei neutraler oder alkalischer Reaktion mit der Dispersion zu vermischen und erst danach anzusäuern. Zum Ansäuern kann jede organische oder anorganische Säure von ausreichender Acidität verwendet werden. Mineralsäuren, insbesondere Phosphorsäure, sind meistens am besten geeignet.

Herkömmliche alkalische Verdickungsmittel enthalten immer Carboxylatgruppen, die beim Auftrocknen eines damit verdickten wäßrigen Systems einen harten Film hinterlassen. Das ist aus anwendungstechnischen Gründen oft unerwünscht. Da viele der erfindungsgemäß eingesetzten Verdickungsmittel zu weichen, geschmeidigen Filmen auftrocknen, kann bereits darin ein Grund ihrer Anwendung liegen.

Ein besonders wichtiges Anwendungsgebiet der Erfindung ist die Verdickung von wäßrigen Kunststoffdispersionen. Sie können aufgrund des Herstellungsverfahrens sauer sein oder werden zum Zwecke der Härtung eines darin enthaltenen Kondensationsharzes sauer eingestellt. Beispiele solcher sauer kondensierbarer Harze sind wasserunlösliche Aminoplastharze und Emulsionspolymerisate, vornehmlich auf Basis von Acryl- oder Methacrylestern, Styrol oder Vinylestern, die Einheiten von Amiden bzw. N-Methylolamiden enthalten. Diese kondensieren im sauren Bereich unter sich oder mit Aminoplastharzen. Auch kationisch emulgierte Harze oder Harze mit kationischen Gruppen können die Anwendung im sauren Bereich erforderlich machen, so daß auch die Verdickung in diesem Bereich wirksam sein muß. Pigmentdruckpasten auf wäßriger Basis sind oft sauer eingestellt und daher erfindungsgemäß verdickbar.

Als Beispiele für weitere wäßrige Systeme, die aus anwendungstechnischen Gründen zur Erleichterung der Handhabung oder zur Verbesserung ihrer Wirksamkeit erfindungsgemäß verdickt werden können, seien anorganische wäßrige Säuren für die Edelstahlbeize, Phosphatierungsbäder, Lötpasten, saure Reinigungsmittellösungen bzw. -pasten, saure Enthaarungsmittel oder Frisiercremes, Kosmetika, Weichspülmittel, Hydrophobierungsmittel, Silikonemulsionen, Kataphoreselacke und Bleichpasten genannt.

## Beispiele

### A). Herstellung der Verdicker-Dispersionen

In einem mit Rührer, Rückflußkühler und Thermometer ausgerüsteten Reaktionsgefäß wird eine Lösung von 1 Gew.-Teil des Umsetzungsproduktes aus 1 Mol Isononylphenol und 100 Mol Äthylenoxid

als Emulgator in 240 Gew.-Teile Wasser auf 80°C erwärmt und mit 0,3 Gew.-Teile des Natriumsalzes der 4,4'-Azobis(4-cyanovaleriansäure) versetzt. Anschließend läßt man innerhalb von 2 Std. eine zuvor aus 360 Gew.-Teile Wasser, 29 Gew.-Teile des oben genannten Emulgators, 0,1 Gew.-Teile des oben genannten Azo-Initiators und den Monomerengemischen hergestellte Emulsion zulaufen. Nach Beendigung des Emulsionszulaufes läßt man noch 1 Std. bei 80°C nachreagieren und kühlt anschließend auf Raumtemperatur ab. Man erhält eine koagulatfreie Dispersion mit einem Feststoffgehalt von ca. 40%.

Zusammensetzung der Monomerengemische:
(In Klammern ist jeweils die Gruppe gemäß Anspruch 1 angegeben, zu der die jeweiligen Monomeren gehören.)

| | | | |
|---|---|---|---|
| 1. | 51,5 | Gew.-Teile | Butylacrylat (B) |
| | 207,2 | Gew.-Teile | Äthylacrylat (B) |
| | 111 | Gew.-Teile | Dimethylaminopropylmethacrylat (A) |
| | 0,3 | Gew.-Teile | Glycoldimethacrylat |
| 2. | 136,9 | Gew.-Teile | Butylacrylat (B) |
| | 81,5 | Gew.-Teile | Äthylacrylat (B) |
| | 111 | Gew.-Teile | Dimethylaminopropylmethacrylat (A) |
| | 37 | Gew.-Teile | 2-Hydroxyäthylmethacrylat (C) |
| 3. | 196,1 | Gew.-Teile | Äthylacrylat (B) |
| | 51,8 | Gew.-Teile | 2-Äthylhexylacrylat (B) |
| | 111 | Gew.-Teile | Dimethylaminopropylacrylat (A) |
| | 11,1 | Gew.-Teile | Methacrylsäure (C) |
| 4. | 55,2 | Gew.-Teile | Butylacrylat (B) |
| | 222 | Gew.-Teile | Äthylacrylat (B) |
| | 92,5 | Gew.-Teile | Dimethylaminoisopropylmethacrylat (A) |
| | 0,3 | Gew.-Teile | Glycoldimethacrylat |
| 5. | 207,2 | Gew.-Teile | Methylacrylat (B) |
| | 33,3 | Gew.-Teile | 2-Äthylhexylacrylat (B) |
| | 129,5 | Gew.-Teile | Diäthylaminopropylacrylat (A) |
| 6. | 129,5 | Gew.-Teile | Methylacrylat (B) |
| | 129,5 | Gew.-Teile | Äthylacrylat (B) |
| | 111 | Gew.-Teile | Dimethylaminobutylmethacrylat (A) |
| 7. | 25,9 | Gew.-Teile | Butylacrylat (B) |
| | 233,1 | Gew.-Teile | Methylacrylat (B) |
| | 111 | Gew.-Teile | 2(4-Morpholinyl)-äthylmethacrylat (A) |

## B). Eigenverdickung

Die Verdickerdispersion wird mit Wasser auf einen Trockengehalt von 5% verdünnt und mit 10%iger Phosphorsäure auf einen pH-Wert von 4,5 eingestellt. Das Gemisch ist anfangs weiß und wird beim Verdicken innerhalb weniger Minuten klar. Die sich ergebende Viskosität wird mit dem Brookfield-Viskosimeter gemessen und ist in der Tabelle angegeben.

## C). Verdickung einer organischen Säure

Zu 100 ml einer 20%igen wäßrigen Propionsäure-Lösung mit einem pH-Wert von 2,4 werden 20 ml der Verdickerdispersionen Nr. 2, 3 und 7 unter Rühren zugegeben. Der pH-Wert steigt auf 3,4 an. Nach 10 Min. erreicht die Viskosität ihren Endwert, der in der Tabelle angegeben ist.

## D). Verdickung von wäßrigen Kunststoffdispersionen

Zwei wäßrige Kunststoffdispersionen A und B werden auf einen Feststoffgehalt von 50 Gew.-% eingestellt und jeweils 100 ml mit 6 g der Verdickerdispersionen Nr. 1, 2, 3 und 7, die mit Wasser auf 20% Feststoffgehalt verdünnt worden sind, versetzt. Anschließend wird mit 3 g 10%iger Phosphorsäure auf pH 2,2 bis 3 angesäuert. Nach 10 Min. wird mittels eines Brookfield-Viskosimeters (Spindel IV,

12 Upm, 20°C) die Viskosität gemessen, die in der Tabelle angegeben ist.

Kunststoffdispersion A: Polymerisatzusammensetzung
Äthylacrylat, Hydroxymethylmethacrylamid, Methacrylamid 92 : 5 : 3 Gew.-Teile,
Viskosität bei 50% Trockengehalt 80 mPa s (Brookfield-Viskosimeter, Spindel I, 30 Upm)
pH-Wert 2,7

Kunststoffdispersion B: Polymerisatzusammensetzung
Butylacrylat, Methylmethacrylat, Hydroxyäthylmethacrylamid, Methacrylamid 42 : 52 : 4 : 2 Gew.-Teile,
Viskosität bei 60% Trockengehalt 2500 mPa s (Brookfield-Viskosimeter, Spindel II, 6 Upm)
pH-Wert 2,2

| Verdicker-Dispersion Nr. | Viskositätswerte (in mPa s) | | | |
|---|---|---|---|---|
| | B. Eigenverdickung | C. Verdickung von wäßr. Propionsäure | D. Verdickung von Kunststoffdisp. | |
| | | | Dispersion A | Dispersion B |
| 1 | 4 200 | – | 35 800 | 51 000 |
| 2 | 370 000 | 210 000 | – | 89 300 |
| 3 | 83 500 | 23 000 | 44 500 | 62 300 |
| 4 | 450 | – | – | – |
| 5 | 1 400 000 | – | – | – |
| 6 | 62 100 | – | – | – |
| 7 | 2 000 | 8 000 | 17 000 | 30 200 |

**Patentansprüche**

1. Verfahren zum Verdicken von wäßrigen Systemen durch Vermischen des wäßrigen Systems mit einer wäßrigen Dispersion eines synthetischen Polymeren und Einstellen auf einen pH-Wert, bei dem das synthetische Polymere wenigstens kolloidal löslich ist, dadurch gekennzeichnet, daß das wäßrige System mit einer wäßrigen Dispersion eines synthetischen Polymeren vermischt wird, das aus

A) 5–100 Gew.-% eines ungesättigten, radikalisch polymerisierbaren Monomeren mit wenigstens einem basischen Stickstoffatom,
B) 0– 95 Gew.-% eines in Wasser höchstens begrenzt löslichen, ungesättigten, radikalisch polymerisierbaren Comonomeren,
C) 0– 30 Gew.-% eines in Wasser löslichen, nicht basischen ungesättigten, radikalisch polymerisierbaren Comonomeren

aufgebaut ist und ein Molekulargewicht von mindestens 500.000 hat und bei einem unter 7 liegenden pH-Wert wenigstens kolloidal löslich ist, ausgenommen Emulsionspolymerisate von 3-Dialkylamino-2,2-dimethylpropylestern einer ungesättigten polymerisierbaren Carbonsäure sowie das Mischpolymerisat aus 30% Dimethylaminoäthylmethacrylat, 30% Äthylacrylat und 40% Methylacrylat.

2. Verfahren nach Anspruch 1, dadurch gekennzeichnet, daß das dispergierte Polymere als Komponente A Einheiten eines Esters einer $\alpha,\beta$-ungesättigten polymerisierbaren Carbonsäure mit wenigstens einem basischen Stickstoffatom enthält, wobei der Alkoholrest des Esters insgesamt wenigstens 5 Kohlenstoffatome enthält.

3. Verfahren nach Anspruch 2, dadurch gekennzeichnet, daß der Ester zwischen dem Ester-Sauerstoffatom und dem basischen Stickstoffatom eine wenigstens 3 Kohlenstoffatome enthaltende Alkylengruppe enthält.

4. Verfahren nach Anspruch 3, dadurch gekennzeichnet, daß die Alkylengruppe wenigstens 3 Kohlenstoffatome in gerader Kette zwischen dem Ester-Sauerstoffatom und dem basischen Stickstoffatom enthält.

5. Verfahren nach den Ansprüchen 1 bis 4, dadurch gekennzeichnet, daß das wäßrige System mit

einer Menge der Dispersion des synthetischen Polymeren vermischt wird, die eine Verdickung um wenigstens 10% bewirkt.

6. Verfahren nach Anspruch 5, dadurch gekennzeichnet, daß die Menge der Dispersion so bemessen wird, daß die Verdickungswirkung, berechnet als das Produkt aus der Anfangsviskosität des wäßrigen Systems und der prozentualen Verdickung, einen Wert von mindestens 1000 [mPa s %] erreicht.

7. Verfahren nach den Ansprüchen 1 bis 6, dadurch gekennzeichnet, daß das wäßrige System auf eine Viskosität von wenigstens 1000 mPa s verdickt wird.

8. Verfahren nach den Ansprüchen 1 bis 7, dadurch gekennzeichnet, daß die wäßrige Dispersion in einer Menge eingesetzt wird, die 0,5—10 Gew.-% des synthetischen Polymeren, bezogen auf den Wasseranteil des wäßrigen Systems, enthält.

9. Verfahren nach den Ansprüchen 1 bis 8, dadurch gekennzeichnet, daß das zu verdickende wäßrige System mindestens 20 Gew.-% Wasser enthält.

## Claims

1. Process for thickening aqueous systems by mixing the aqueous system with an aqueous dispersion of a synthetic polymer and adjusting to a pH value at which the synthetic polymer is at least colloidally soluble, characterised in that the aqueous system is mixed with an aqueous dispersion of a synthetic polymer which is composed of

A) 5—100% by weight of an unsaturated radically polymerisable monomer with at least one basic nitrogen atom,
B) 0—95% by weight of an unsaturated radically polymerisable comonomer which has at most limited solubility in water,
C) 0—30% by weight of a water-soluble non-basic unsaturated radically polymerisable comonomer

and has a molecular weight of at least 500,000 and is at least colloidally soluble at a pH of below 7, with the exception of emulsion polymers of 3-dialkylamino-2,2-dimethylpropyl esters of an unsaturated polymerisable carboxylic acid and the copolymer of 30% dimethylaminoethylmethacrylate, 30% ethylacrylate and 40% methylacrylate.

2. Process as claimed in claim 1, characterised in that the dispersed polymer as component A contains units of an ester of an $\alpha,\beta$-unsaturated polymerisable carboxylic acid with at least one basic nitrogen atom, whilst the alcohol group of the ester contains a total of at least 5 carbon atoms.

3. Process as claimed in claim 2, characterised in that the ester contains, between the ester oxygen atom and the basic nitrogen atom, an alkylene group containing at least 3 carbon atoms.

4. Process as claimed in claim 3, characterised in that the alkylene group contains at least 3 carbon atoms in a straight chain between the ester oxygen atom and the basic nitrogen atom.

5. Process as claimed in claims 1 to 4, characterised in that the aqueous system is mixed with a quantity of the dispersion of the synthetic polymer which brings about a thickening of at least 10%.

6. Process as claimed in claim 5, characterised in that the quantity of the dispersion is such that the thickening effect, calculated as the product of the initial viscosity of the aqueous system and the percentage thickening, attains a value of at least 1000 (mPa s %).

7. Process as claimed in claims 1 to 6, characterised in that the aqueous system is thickened to a viscosity of at least 1000 mPa s.

8. Process as claimed in claims 1 to 7, characterised in that the aqueous dispersion is used in a quantity which contains 0.5—10% by weight of the synthetic polymer, based on the water content of the aqueous system.

9. Process as claimed in claims 1 to 8, characterised in that the aqueous system which is to be thickened contains at least 20% by weight of water.

## Revendications

1. Procédé pour épaissir des systèmes aqueux par mélange du système aqueux avec une dispersion aqueuse d'un polymère synthétique et réglage à un pH auquel le polymère synthétique est soluble au moins à l'état colloïdal, caractérisé en ce que, on mélange le système aqueux avec une dispersion aqueuse d'un polymère synthétique qui est constitué de:

A) 5 à 100% en poids d'un monomère insaturé apte à la polymérisation radicalaire portant au moins un atome d'azote basique,
B) 0 à 95% en poids d'un comonomère insaturé, apte à la polymérisation radicalaire, ayant une solubilité au maximum limitée dans l'eau,
C) 0 à 30% en poids d'un comonomère insaturé non basique soluble dans l'eau, apte à la polymérisation radicalaire,

et qui présente un poids moléculaire d'au moins 500.000 et est soluble au moins à l'état colloïdal à un pH inférieur à 7, à l'exception des polymères en émulsion d'esters 3-dialkylamino-2,2-diméthylpropyliques d'un acide carboxylique insaturé polymérisable et du copolymère de 30% de méthacrylate de diméthylaminoéthyle, 30% d'acrylate d'éthyle et 40% d'acrylate de méthyle.

2. Procédé selon la revendication 1, caractérisé en ce que le polymère en dispersion contient en tant que composant A des motifs d'un ester d'un acide carboxylique alpha, bêta-insaturé polymérisable portant au moins un atome d'azote basique, le reste alcoolique de l'ester contenant au total au moins 5 atomes de carbone.

3. Procédé selon la revendication 2, caractérisé en ce que l'ester contient un groupe alkylène à au moins 3 atomes de carbone entre l'atome d'oxygène d'ester et l'atome d'azote basique.

4. Procédé selon la revendication 3, caracterisé en ce que le groupe alkylène contient au moins 3 atomes de carbone en chaîne droite entre l'atome d'oxygène d'ester et l'atome d'azote basique.

5. Procédé selon les revendications 1 à 4, caractérisé en ce que le système aqueux est mélangé avec la dispersion du polymère synthétique en quantité provoquant un épaississement d'au moins 10%.

6. Procédé selon la revendication 5, caractérisé en ce que la quantité de la dispersion est réglée de manière que l'effet épaississant, exprimé par le produit de la viscosité initiale du système aqueux par l'épaississement %, atteint une valeur d'au moins 1.000 mPa.s %.

7. Procédé selon les revendications 1 à 6, caractérisé en ce que le système aqueux est épaissi à une viscosité d'au moins 1.000 mPa.s.

8. Procédé selon les revendications 1 à 7, caractérisé en ce que la dispersion aqueuse est utilisée en quantités contenant de 0,5 à 10% en poids du polymère synthétique par rapport à l'eau contenue dans le système aqueux.

9. Procédé selon les revendications 1 à 8, caractérisé en ce que le système aqueux à épaissir contient au moins 20% en poids d'eau.